Europäisches Patentamt

European Patent Office

Office européen des brevets

(19)

(11) Veröffentlichungsnummer : **0 160 129**
**B1**

(12) # EUROPÄISCHE PATENTSCHRIFT

(45) Veröffentlichungstag der Patentschrift :
01.02.89

(21) Anmeldenummer : 84116217.5

(22) Anmeldetag : 22.12.84

(51) Int. Cl.⁴ : **A 61 B 5/10**, A 61 H 1/00,
A 63 B 21/00

(54) **Vorrichtung zur Erfassung und Verbesserung der Funktionsfähigkeit des Bewegungsapparates des Menschen.**

(30) Priorität : 06.04.84 DE 3413063
29.11.84 DE 3443512

(43) Veröffentlichungstag der Anmeldung :
06.11.85 Patentblatt 85/45

(45) Bekanntmachung des Hinweises auf die Patenterteilung : 01.02.89 Patentblatt 89/05

(84) Benannte Vertragsstaaten :
AT BE CH DE FR GB IT LI LU NL SE

(56) Entgegenhaltungen :
EP-A- 0 041 807
EP-A- 0 092 541
WO-A-83 /020 52
CH-A- 83 213
DE-A- 1 478 056
DE-C- 198 212
FR-A- 2 459 040
FR-A- 2 481 599
US-A- 4 136 682
US-A- 4 306 571

(73) Patentinhaber : GGT Medizin-Electronic-Systeme
GmbH
Badstrasse 7
D-8000 München 70 (DE)

(72) Erfinder : Ott, Emil, Dipl. Ing.
Wiebekingstrasse 2a
D-8000 München 50 (DE)
Erfinder : König, Herbert, Prof. Dr.-Ing.
Simmernstrasse 5
D-8000 München 40 (DE)

(74) Vertreter : Hansmann, Dierk, Dipl.-Ing.
Jessenstrasse 4
D-2000 Hamburg 50 (DE)

## Beschreibung

Die Erfindung bezieht sich auf eine Vorrichtung zur Erfassung und Verbesserung der Funktionsfähigkeit des Bewegungsapparates von Menschen, insbesondere seiner Extremitäten, mit einer der Aufnahme der Extremität dienende Haltevorrichtung.

Es besteht das Problem, die Funktionsfähigkeit des Bewegungsapparates des Menschen nach objektiven Kriterien und die quantitative Erfassung des Grades der Funktionsfähigkeit, sowohl beim kranken als auch beim gesunden Menschen zu erfassen. Zwar ist durch die Elektromyographie eine Untersuchungsmethode geschaffen worden, die mittelbar einen Schluß auf die Funktionsfähigkeit bzw. Beweglichkeit von Gliedmaßen zuläßt, sie erlaubt jedoch bei bestimmten Arten von Funktionsstörungen von Gliedmaßen keine Aussage über den Grad des Defektes.

Darüberhinaus ist ebenfalls die Verbesserung der Funktionsfähigkeit eines als defekt erkannten Bewegungsapparates unter Festlegung quantitativer Bewegungsparameter ebenfalls ein Problem. Ein physikalisches Training, beispielsweise erkrankter Gliedmaßen, wird häufig durch Menschen ausgeführt, jedoch weist dieses ausgeführte Training der Gliedmaßen den Nachteil auf, daß die Bewegung niemals unter beliebig oft nach Art und Größe reproduzierbaren Bewegungskriterien vonstatten gehen kann. Schließlich erfordern Bewegungsabläufe zur Verbesserung der Funktionsfähigkeit der Gliedmaßen einen über längere Zeit zu erbringenden erheblichen Kraftaufwand, so daß auch aus diesem Grunde der Durchführung Grenzen gesetzt sind.

Eine mechanische Anordnung zur Verbesserung der Funktionsfähigkeit ist nach der FR-A-2 459 040 bekannt geworden. Hierbei wird durch eine passive Vorrichtung ein Bewegungswinkel eingestellt und erfaßt, wobei über einen Schrittmotor eine Verstellung einer Meßplatine erfolgt. Es besteht der Mangel, daß die Extremität selbst von dem Probanden ohne Beeinflußung des Bewegungsablaufes zu bewegen ist und kein Drehmoment in der Gelenkachse messbar ist, so daß keine Rückschlüsse auf den vorliegenden Zustand möglich sind.

Ferner ist eine Vorrichtung gemäß DE-A-1 478 056 zum Muskeltraining bekannt geworden, die sich auf eine andere Gattung von Vorrichtungen bezieht und nicht zur Messung der Funktionsfähigkeit von Extremitäten herangezogen wird.

Es ist Aufgabe der Erfindung, eine Vorrichtung zu schaffen, die es ermöglicht, für diagnostische und Testzwecke die Funktionsfähigkeit des Bewegungsapparates des Menschen sowohl kranker als auch gesunder Menschen anhand objektiver und qualitativer Bewegungsparameter zu ermitteln sowie auch Bewegungsabläufe über langandauernde Zeiträume unter Beachtung beliebig reproduzierbarer qualitativer und quantitativer Betriebsparameter zu gewährleisten.

Gelöst wird die Aufgabe dadurch, daß die Haltevorrichtung über einen Antriebsmotor um eine in der Haltevorrichtung aufgenommene Gelenkachse einer Extremität entsprechenden Achse drehbar ist und die Antriebsachse mit einer Meßwinkelaufnehmereinrichtun sowie einer Einrichtung zur Aufnahme des Drehmomentes versehen ist.

Der Vorteil besteht darin, daß die Haltevorrichtung mit der darin angeordneten menschlichen Extremität mit einem von außen aufgebrachten Drehmoment in Bezug auf seine Größe variabel einstellbar ist und somit die Extremität aktiv verdrehbar ist sowie alle gewünschten Dreh- bzw. Bewegungsgrade durchlaufen kann, wozu andere Anordnungen, wie entsprechend der DE-A-29 12 981 nicht in der Lage ist, da dort die zu untersuchenden Extremitäten lediglich feste Blattfedereinrichtungen zur Ermittlung bestimmter Bewegungsgrößen der Extremität verbiegen und der Verbiegungsgrad entsprechend gemessen und angezeigt wird.

Weiterhin besteht der Vorteil, daß die Haltevorrichtung selbst bei Aufnahme so unterschiedlicher Extremitäten wie Arme und Beine grundsätzlich den gleichen Aufbau hat, was wegen der Niedrighaltung der Kosten für Herstellung, Ersatzteillagerhaltung und Wartung erhebliche Vorteile hat. Darüberhinaus ist auch die Steuerung des Antriebes der Vorrichtung beim Einsatz, beispielsweise zur Bewegung des Beines eines Menschen und zur Bewegung eines Armes grundsätzlich die gleiche, so daß auch hier keine zusätzlichen konstruktiven Maßnahmen nötig sind.

Eine vorteilhafte Ausbildung besteht darin, daß der Antriebsmotor ein Schrittmotor ist, der über ein Getriebe mit der Achse der Haltevorrichtung verbunden ist. Hierbei besteht der Vorteil, daß bei Ausfall von Steuereinrichtungen kein Hochlaufen des Antriebsmotors erfolgt, wie dies beispielsweise bei einer Regelung in Verbindung mit einem Gleichstrommotor geschehen könnte, mit den sich daraus ergebenden nachteiligen Folgen für die zu untersuchende Person und die Vorrichtung selbst.

Vorzugsweise ist zwischen Getriebe und der Achse der Haltevorrichtung eine Kupplungseinrichtung angeordnet, die bei einem frei wählbaren höchstzulässigen Drehmoment auslösbar ist.

Obwohl grundsätzlich die Achse des Antriebsmotors mit oder ohne Getriebe direkt mit der Achse der Haltevorrichtung verbunden sein kann, ist gemäß einer weiteren vorteilhaften Ausführungsform der Erfindung vorgesehen, daß die Achse der Haltevorrichtung mit der Antriebsachse über eine Zahnradanordnung und unter Zwischenschaltung eines Zahnriemens miteinander verbunden ist. Eine derartige Ausbildung der Vorrichtung ermöglicht auf einfache Weise über das Übersetzungsverhältnis Einfluß auf die nötige Motorleistung zu nehmen und gestattet es, den Antriebsmotor unterhalb der Achse bzw. unterhalb der Haltevorrichtung insgesamt anzubringen, so daß der eigentliche Antrieb der Vorrich-

tung für die zu untersuchende Person nicht sichtbar angeordnet ist und damit der Antriebsmotor nicht hinderlich in den Bereich der Bedienungsperson bzw. in den der zu untersuchenden Person hineinragt.

Die Antriebsachse ist mit einer Meßwinkelaufnahmeeinrichtung sowie einer Einrichtung zur Aufnahme des Drehmomentes versehen. Die gesonderte Meßwinkelaufnahmeeinrichtung gestattet die Feststellung selbst kleinster Winkeländerungen, beispielsweise bis zu einer Winkelauflösung von 2 Zehntel Grad und darunter. Die Einrichtung zur Aufnahme des Drehmomentes und dessen Darstellung in Abhängigkeit vom Drehwinkel erlaubt eine schnelle Evaluation des Zustandes der zu untersuchenden Person und dient ebenfalls der Erfassung des Drehmomentes für gesonderte theoretische Betrachtungen, wenn beispielsweise durch Integration die Muskelverspannarbeit bzw. Leistung berechnet werden soll.

Ferner ist vorgesehen, daß der Drehwinkel der Haltevorrichtung über den Antriebsmotor steuernde Taktimpulse erfaßbar ist. Zur weiteren Verbesserung wird vorgeschlagen, daß eine den tatsächlichen Drehwinkel des Gelenks einer Extremität erfassende Winkelaufnehmereinrichtung unmittelbar auf der Extremität angebracht ist.

Weiterhin wird zur einfachen Einstellung vorgesehen, daß die Bewegung der Haltevorrichtung über den Antriebsmotor durch einen zugeordneten Rechner in Abhängigkeit programmierbarer Bewegungs- und Drehmomentsparameter steuerbar ist.

Mit der Vorrichtung werden vorzugsweise die bei der Bewegung der Haltevorrichtung um ihre Achse auftretenden Kräfte durch Reibung, Schwerkraft und Trägheit durch den Rechner erfaßt und nachfolgend zur Darstellung der für die Bewegung der Haltevorrichtung typischen tatsächlichen Bewegungsparameter einer Extremität minimalisiert. Die ermittelten Eichparameter werden somit zur Korrektur der nachfolgend bei einer Diagnose ermittelten Meßwerte herangezogen, so daß die tatsächlichen Bewegungsparameter zur Beurteilung und zur datenmäßigen Weiterverarbeitung zur Verfügung stehen.

Zur einfachen Beurteilung wird vorgeschlagen, daß die ermittelten Bewegungsparameter wie das Drehmoment der Haltevorrichtung als Funktion dieses Verdrehungswinkels, der zeitabhängige Verdrehungswinkel als Funktion eines Drehimpulses sowie die Verdrehungsarbeit und Leistung ermittelbar und über Aufzeichnungsmittel aufzeichenbar sind.

Zur umfassenden Beurteilung wird vorgeschlagen, daß die Bewegungsparameter für jede Drehrichtung der Haltevorrichtung gesondert ermittelbar und über Aufzeichnungsmittel aufzeichenbar sind.

Schließlich ist vorgesehen, daß die ermittelten Bewegungsparameter durch Anzeigemittel darstellbar sind.

Weitere meßspezifische Ausgestaltungen der Erfindung sind durch die Merkmale der Unteransprüche 13 bis 15 gekennzeichnet.

Die Erfindung wird nun unter Bezugnahme auf die schematischen Zeichnungen anhand eines Ausführungsbeispieles eingehend beschrieben. Darin zeigen :

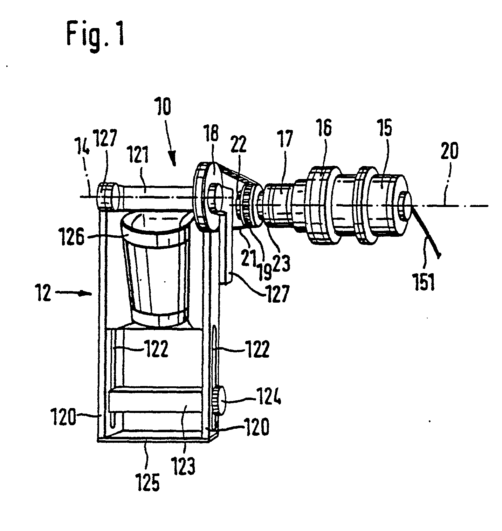

Fig. 1 in perspektivischer Darstellung eine Vorrichtung zur Aufnahme einer Extremität in Form eines menschlichen Armes,

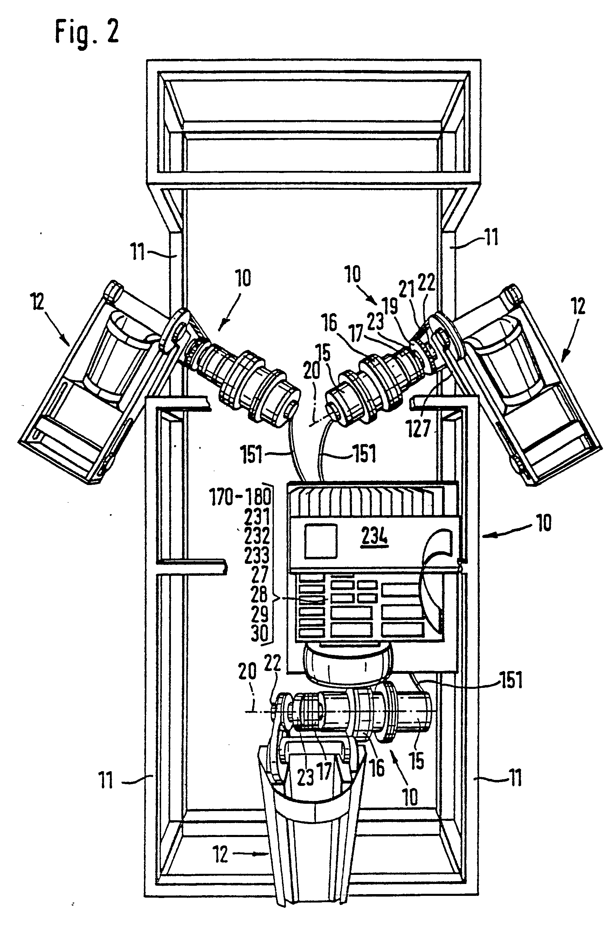

Fig. 2 in perspektivischer Darstellung eine Draufsicht auf ein Gestell mit zwei Vorrichtungen zur Aufnahme menschlicher Arme und einer Vorrichtung zur Aufnahme eines menschlichen Beines unter Weglassung einer Ruhefläche für den Menschen,

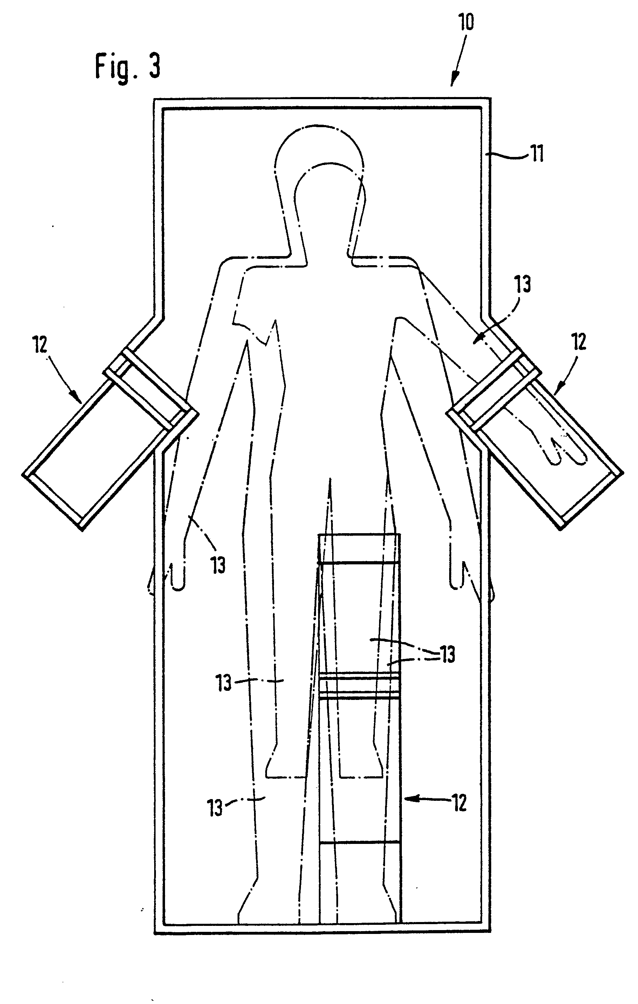

Fig. 3 eine Draufsicht auf das in Fig. 2 dargestellte Gestell mit umrißartig dargestellten Menschen unterschiedlicher körperlicher Größe mit teilweise in der Haltevorrichtung angeordneten Extremitäten,

Fig. 4 ein Blockschaltbild der Vorrichtung zur Darstellung der Steuerungsfunktionszusammenhänge,

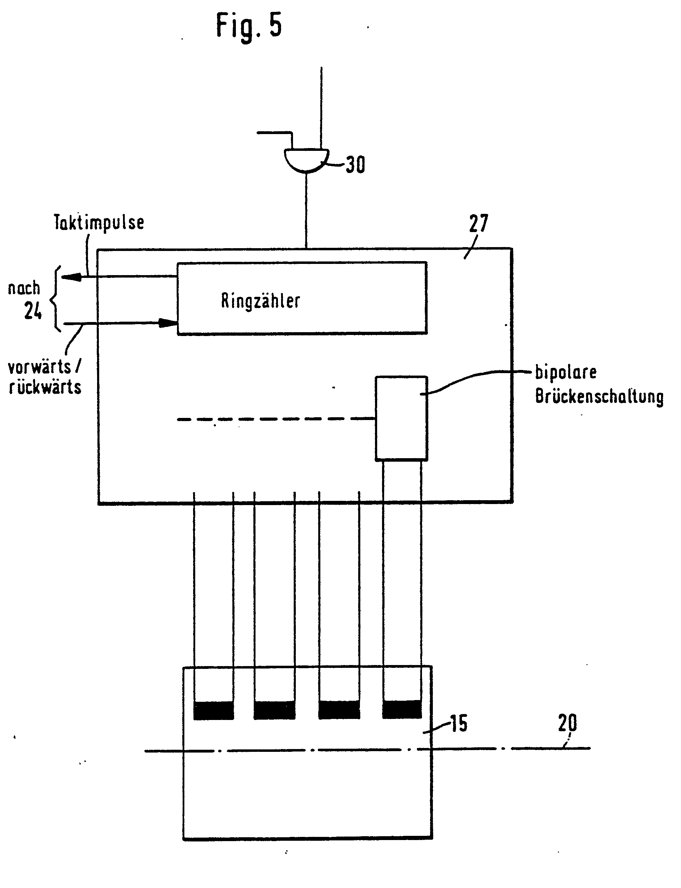

Fig. 5 ein Blockschaltbild der Ansteuerungselektronikeinheit und

Fig. 6 ein Schaltbild der Steuerschaltung der Kupplungseinrichtung.

Der mechanische Teil der Vorrichtung 10 besteht im wesentlichen aus einer Haltevorrichtung 12, die der Aufnahme einer Extremität 13 dient. Die Haltevorrichtung 12 weist eine geeignet ausgebildet Achse 14 auf, um die diese schwenkbar ist.

Die Haltevorrichtung 12 umfaßt zwei im wesentlichen parallel zueinander angeordnete Stege 120, die über einen u-förmig ausgebildeten Quersteg 121 miteinander auf der einen Seite verbunden sind. Durch die Schenkel des u-förmig ausgebildeten Querstegs 121 geht die Achse 14 der Haltevorrichtung 12 hindurch. Die freien Enden der Stege 120 weisen Schlitze 122 auf, in denen ein Handgriff 123 verschiebbar und durch Feststellmittel 124 feststellbar angeordnet ist. Durch eine Abschlußplatte 125 wird die Haltevorrichtung 12 im wesentlichen parallel zu ihrer Achse 14 an ihrem freien Ende abgeschlossen. Zwischen Quersteg 121 und dem zur Achse 14 weisenden Ende der Schlitze 122 ist eine Einrichtung 126 zum Festhalten der Extremität 13 angeordnet. Die Einrichtung 126 ist entsprechend der Größe und Art der Extremität 13 ausgebildet, die in ihr aufgenommen werden soll, beispielsweise in Form eines Armes oder eines Beines. Lagereinrichtungen 127 zu beiden Seiten der verschwenkbaren Querstrebe der Haltevorrichtung 12 sind auf einer Unterlage, beispielsweise in Form eines Gestells 11, geeignet befestigt.

Unterhalb der Achse 14 ist im wesentlichen parallel zu dieser Achse ein Antriebsmotor 15 angeordnet, der mit einem Getriebe 16, einer Kupplungseinrichtung 17 und einem Zahnrad 19 versehen ist. Über einen Zahnriemen 21 treibt der Antriebsmotor 15 ein Zahnrad 18 an, das auf der Achse 14 der Haltevorrichtung 12 angeordnet ist. Durch diese Anordnung der durch den Antriebsmotor 15, das Getriebe 16 und die Kupplungseinrichtung 17 und das Zahnrad 19 hindurchgehenden Antriebsachse 20 im wesentlichen unterhalb der Achse 14 ist es möglich, das liegeartige Gestell 11 frei von wesentlichen Teilen der Vorrichtung 10 zu halten, so daß nachteilige psychologische Wirkungen auf die Testperson bzw. den Patienten nicht befürchtet zu werden brauchen.

Auf der Antriebsachse 20 ist ebenfalls auf geeignete Weise eine Meßwinkelaufnehmeeinrichtung 22 in Form eines Winkeldekoders angeordnet, der elektrische Impulse liefert, die von der nachfolgend noch beschriebenen elektronischen Steuerung erfaßt und ausgewertet werden. Die zweikanaligen Impulse (2 Kanal Vor-Rückwärtserkennung + 1 Indeximpuls) werden je nach Phasenlage zueinander vor- und rückwärts gezählt, so daß dadurch ein absoluter Zählerstand in einem noch später beschriebenen Rechner 24 existiert. Der Winkeldekoder besteht aus einer « 500 - Loch - Scheibe », wobei durch eine Vierfachauswertung somit 2 000 Stellungen pro Umdrehungen unterschieden werden können, woraus eine Auflösung von 0,18° resultiert.

Zwischen Zahnrad 19 und Kupplungseinrichtung 17 ist eine Einrichtung 23 zur Aufnahme des Drehmoments angeordnet. Die Einrichtung 23 ist in Form eines Dehnungs-Meßstreifenaufnehmers ausgebildet. Bei Verdrehung der Antriebsachse 20 beim Antrieb der Haltevorrichtung 12 erfolgt bei einem entgegen der Drehung des Antriebsmotors gerichteten Drehmoment eine Verdrehung der Einrichtung 23 relativ zur Antriebsachse 20 des Antriebsmotors 15, in dem ein Drehmomentaufnehmer, der hier in Form eines Biegebalkenaufnehmers ausgebildet ist, der Teil der Einrichtung 23 ist, unter dem Einfluß der durch das Gegendrehmoment auf ihn ausgeübten Kraft verbogen wird. Das Verbiegen wird durch die Dehnungsmeßstreifen in eine Widerstandsänderung umgesetzt und diese wiederum in einer Brückenschaltung in eine Spannungsänderung, wobei die durch das Gegendrehmoment ausgeübte Kraft direkt proportional der Spannungsänderung ist.

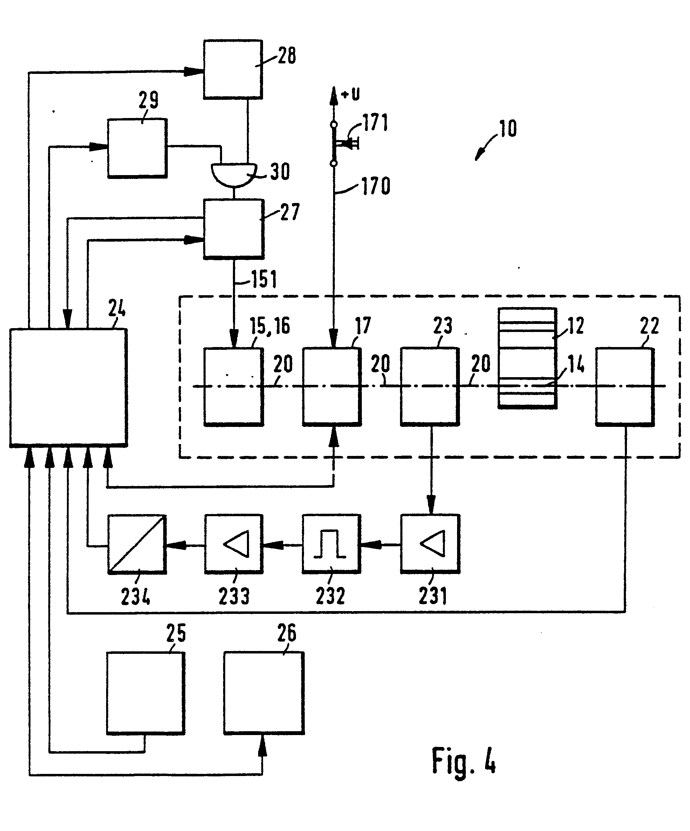

Die Vorrichtung 10 umfaßt eine Mehrzahl von Einrichtungen, die der Steuerung der Haltevorrichtung 12 dienen, vgl. Fig. 4. Die Vorrichtung 10 weist einen Rechner 24 auf, der sowohl in Form eines nur diese eine Vorrichtung steuernden Mikroprozessors als auch in Form eines handelsüblichen Prozeßrechners ausgebildet sein kann. Der Rechner 24 ist über eine Ansteuerelektronik 27 mit dem Antriebsmotor verbunden. Ebenfalls mit dem Rechner verbunden ist die Kupplungseinrichtung 17, die ebenfalls von außen durch die Bedienungsperson oder die zu untersuchende Person deaktivierbar ist, was symbolisch durch die Leitung 170 dargestellt ist und nachfolgend noch beschrieben wird. Die zuvor schon beschriebene Einrichtung 23 zur Aufnahme des Drehmoments ist über einen Verstärker 231, ein Tiefpaßfilter 232, einen Verstärker 233 und einen Analog-Digital-Wandler 234 mit dem Rechner 24 verbunden. Das von der Einrichtung 23 kommende Signal wird im Verstärker 231 verstärkt und in dem nachfolgend angeordneten Tiefpaßfilter 232 geglättet. Die Eckfrequenz des Tiefpaßfilters 232 verläuft direkt proportional der Geschwindigkeit des Antriebsmotors 15. Dadurch werden höherfrequente Anteile, die keine Aussagen zur gewünschten Messung der Bewegungsparameter liefern, beseitigt. Die Frequenz dieser Störungen, hervorgerufen durch mechanische Resonanzen und Schwingungen der Vorrichtung 10 oder gegebenenfalls des gesamten Gestells 11, sind geschwindigkeitsabhängig, d. h. durch die Verknüpfung der Eckfrequenz des Tiefpaßfilters 232 mit der Umdrehungsgeschwindigkeit des Antriebsmotors 15 erreicht man automatische die fehlerfreien Meßwertaufnahmen im richtigen Frequenzbereich. Nachfolgend wird das Signal durch den Verstärker 233 zur Pufferung des diesem nachfolgenden Analog-Digital-Wandlers 234 gepuffert. Der Erfassungs- bzw. Meßbereich der Einrichtung 23 zur Aufnahme des Drehmoments beträgt im vorliegenden Fall ± 120 Nm. Dieser Meßbereich wird durch den im vorliegenden Fall beschriebenen 12 bit Analog-Digital-Wandler 234 so digitalisiert, daß die kleinste Auflösung 0,06 Nm beträgt.

Schließlich ist die zuvor schon erwähnte Meßwinkelaufnehmeeinrichtung 22, die ebenfalls digitale Signale liefert, mit dem Rechner 24 verbunden. Dieser Winkeldecoder 22 dient eigentlich nur zur Kontrolle der Stellung der Extremität. Außerdem kann die Stellung der Extremität bei ausgekuppeltem Antrieb gemessen werden (z. B. : notwendig bei Aufnahme der Winkelstellung für Therapiebewegung). Bei der eigentlichen Messung (= Diagnose) wird die Winkelstellung durch ein Mitzählen der Motorimpulse gewonnen. (Bei 120° Bewegung werden an die Motorsteuerung ca. 16.000 Impulse gegeben - d. h. die maximale Auflösung beträgt : 120°/16 000 = 0,0075°). Dies ist ein weiterer Vorteil der Verwendung des Schrittmotors 15.

Die Ansteuerelektronikeinrichtung 27 setzt eine ankommende Impulsfolge in die notwendigen Stromzustände der einzelnen 4 Phasen des Schrittmotors 15 um. Diese einzelnen Zustände sind einem Ringzähler zugeordnet, der bei jedem Impuls (abhängig vom Eingang vorwärts/rückwärts) um eine Stellung weiterschaltet. Der Strom einer einzelnen Phase wird von einer gechoppten bipolaren Brückschaltung erzeugt.

Mit der Ansteuerungselektronikeinrichtung 27 ist ein Bit-Rate-Multitlier 28 verbunden, der wiederum vom Rechner 24 gesteuert wird. Im Bit-Rate-Multitlier 28 wird vom Rechner 24 gesteuert eine Frequenzfolge erzeugt, die wiederum zur Bewegung des Antriebsmotors 15 dient, wie es zuvor im Zusammenhang mit der Darstellung der Funktion der Ansteuerungselektronikeinrichtung 27 beschrieben worden ist. Es können Frequenzen von 0 bis 8 000 Hz mit einer Auflösung von 2 Hz erzeugt werden.

Der Rechner muß, um die Taktfolge für den Antriebsmotor 15 aufrecht zu erhalten, eine ständige Aktion (jede 1/100 sek.) durchführen, d. h. ein Monoflop 29 muß ständig vom Rechner 24 getriggert werden. Wird diese Bedingung nicht eingehalten, beispielsweise bei evtl. Ausfall des Rechners 24, wird die Frequenzfolge durch das Gatter 30 unterbrochen und der Antriebsmotor 15 gestoppt sofort. Die Bedienungsperson oder ggf. die zu untersuchende bzw. zu behandelnde Person kann dann die Kupplung 17 durch einen Not-Aus-Schalter 171 öffnen.

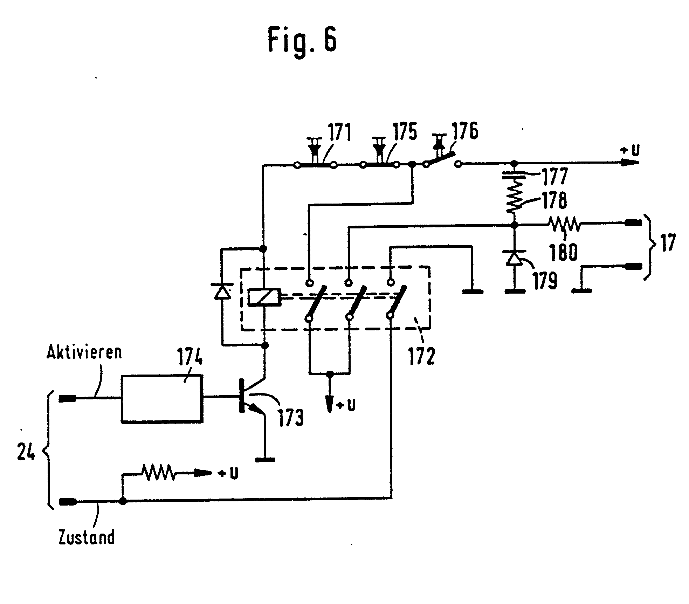

Die der Kupplungseinrichtung 17 zugeordnete Steuerschaltung ist in Fig. 6 dargestellt. Sie besteht aus einem Relais 172 mit drei getrennten Schließkontakten einem Transistor 173, einem Optokoppler 174 und neben dem Öffner 171 aus einem weiteren Öffner 175 sowie einem Schließer 176. Vom Rechner 24 erhält der Optokoppler 174 ein Signal, so daß der Transistor 173 leitend wird. Sind die beiden Öffner 171 (Not-Aus-Öffner) und der Öffner 175 geschlossen, und wird der Taster 176 gedrückt, so zieht das Relais 172 an, da der Stromkreis über die positive Versorgungsspannung, den Taster 176, die Öffner 175 und 171 über die Spule des Relais 172 und über den Transistor 173 zur Masse geschlossen ist. Die Schalter des Relais werden nachfolgend geschlossen und das Relais 172 hält sich selbst. Der Taster 176 kann wieder losgelassen werden. Die Kupplungseinrichtung 17 kann nun über die an der Ausgangsklemme der Steuerschaltung liegende positive Versorgungsspannung, da es sich um eine Magnetkupplung handelt, geschlossen werden. Die zwischen der positiven Versorgungsspannung und der zur Kupplungseinrichtung 17 führenden Ausgangsklemme der Steuerschaltung geschaltete Reihenschaltung aus Kondensator 177 und Widerstand 178 dient zur Glättung bzw. Entstörung der Schaltung, ebenso wie die Diode 179 zwischen der zur Kupplungseinrichtung 17 führenden Ausgangsklemme der Steuerschaltung und der Masse. Der Rechner 24 kann über eine von der Masse über einen Kontakt des Relais 172 geschalteten Fühlerleitung den momentanen Zustand der Kupplungseinrichtung 17 überwachen, beispielsweise wenn durch Drücken des Öffners 171 (Not-Aus-Schalter) die Kupplung deaktiviert worden ist oder zur Überprüfung der Deaktivierung der Kupplungseinrichtung 17 über den Optokoppler 174 und den Transistor 173 durch den Rechner 24 selbst. Die Kupplungseinrichtung 17 bleibt nun solange deaktiviert, bis der Schließer 176 gedrückt worden ist, unter der Voraussetzung, daß der Rechner 27 über Optokoppler 174 den Transistor 173 leitend schaltet.

An den Rechner 24 ist noch ein Aufzeichnungsmittel 25 in Form eines Druckers, Plotters oder in Form eines Koordinatenschreibers angeschlossen, sowie ein Anzeigemittel 26 in Form eines Sichtgerätes.

Nach dem Betten einer Person auf einer Unterlage auf dem Gestell 11 wird eine oder werden mehrere Extremitäten 13 in die jeweilige Haltevorrichtung 12 der Vorrichtung bzw. Vorrichtungen 10 eingelegt und dort mittels der Einrichtung 126 festgehalten.

Von der Bedienungsperson werden nun über eine dem Rechner 24 zugeordnete Eingabetastatur neben dem Datum der Uhrzeit, der Bezeichnung der Person und der Bedienungsperson und einem evtl. Kommentar, die bewegungsspezifischen Parameter wie Bewegungsdauer, Geschwindigkeit, Beschleunigung, Winkelbereich und maximal einzuhaltendes Drehmoment sowie Angaben zur Formatierung der ermittelten Werte bei der Darstellung im Aufzeichnungsmittel 25 bzw. im Anzeigemittel 26 eingegeben. Vor der eigentlichen Aufnahme der Extremität 13 in der Vorrichtung 10 erfolgt jedoch nach jedem Neu-Einschalten des Geräts, beispielsweise einmal am Tag, die Aufnahme einer Eichkurve der Vorrichtung, d. h. es werden die die Reibung in den Lagern der Haltevorrichtung 12, die durch das Gewicht der Haltevorrichtung 12 und die durch seine Trägheit verursachten Meßfehler verursachenden Parameter mit der leeren Haltevorrichtung 12 ermittelt, so daß diese nachfolgend bei der Darstellung der für die Bewegung der Haltevorrichtung 12 mit eingelegter Extremität 13 typischen Bewegungsparameter berücksichtigt werden können, indem sie minimalisiert werden.

Nach Eingabe der Parameter in den Rechner 24 wird die Kupplungseinrichtung 17 aktiviert und die Haltevorrichtung 12 wird beispielsweise auf « Meßbereich Anfang Streckung » positioniert. Nachfolgend erfolgt die Messung « Streckung ». Daran anschließen kann beispielsweise ein Positionieren auf « Meßbereich Anfang Beugung » erfolgen und nachfolgend die Messung « Beugung ». Anschließend wird die Kupplung deaktiviert. Nachfolgend erscheint beispielsweise der oder die ermittelten Meßwerte, beispielsweise in Form eines auf einem Plotter dargestellten Diagramms. Dieser für einen Diagnostikfall typische Verfahrensablauf kann anschließend beliebig oft unter Variation einzelner Bewegungsparameter wiederholt werden und erlaubt somit eine objektive und quantitative beliebig oft reproduzierbare Erfassung des Grades der Funktionsfähigkeit des jeweils untersuchten Bewegungsapparates eines Menschen.

Für therapeutische Zwecke vollzieht sich die Eingabe der Parameter in den Rechner 24 auf gleiche Weise und nachdem diese abgeschlossen ist, vollführt die Vorrichtung mit in der Haltevorrichtung 12 eingelegter Extremität 13 die den eingegebenen Befehlen entsprechenden Bewegungsabläufe. Zu diesem Zweck ist es möglich, daß der Bewegungsablauf für therapeutische oder Trainingszwecke anhand eines bestimmten vorgegebenen Programms durchgeführt wird, es ist jedoch auch mögl-ich, daß die Bedienungsperson die von der Vorrichtung 10 nachfolgend automatisch zu durchlaufenden Bewegungszyklen zunächst durch Bewegung der Haltevorrichtung 12 in Echtzeit vorgibt und der Rechner dabei die durch die Bedienungsperson geführte Bewegung der Haltevorrichtung in Bezug auf Beschleunigung und Geschwindigkeit analysiert und speichert, so daß die Vorrichtung 10 anhand dieser Werte die Bewegung bei der Therapie selbständig oft ausführen kann.

Schließlich besteht noch die Möglichkeit einer Verknüpfung von Therapie und Diagnose, indem beispielsweise zunächst die Parameter einer Person für diagnostische Zwecke gemessen werden, nachfolgend dann eine bestimmte Anzahl von Therapiebewegungen durchgeführt wird, und dann anschließend wiederum eine Messung der Person zu diagnostischen Zwecken erfolgt und schließlich nachfolgend die Auswertung durch den Rechner 24. Darüberhinaus sind beliebige andere Kombinationen von Therapie- und Diagnoseschritten mit dem hier beschriebenen Verfahren und der Vorrichtung 10 möglich, je nach der Art der Funktionsstörung des Bewegungsapparates. Sowohl beim Einsatz der Vorrichtung 10 für diagnostische Zwecke als auch zum Zwecke der Therapie wird vom Rechner 24 ständig das von der Bedienungsperson eingestellte maximale Drehmoment der Haltevorrichtung 12, seine Winkelstellung, das Auslösen einer Notsituation durch Drücken einer der beiden Öffner 171 und 174 und das Auslösen einer Notsituation durch Eingabe beliebiger Tasten am Terminal überwacht.

Vom Rechner 24 wird nach der Ermittlung der entsprechenden Bewegungsparameter das maximal vorkommende Drehmoment für Beugung und Streckung, die Drehmomentarbeit für Beugung und Streckung, die Hysterese (Arbeit Beugung — Arbeit Streckung) und die Leistung für Beugung und Streckung berechnet. Diese Werte weren vorzugsweise im Klartext auf einem Aufzeichnungsmittel 25, beispielsweise einem Plotter dargestellt. Ebenfalls dargestellt werden auf dem Aufzeichnungsmittel 25 in grafischer Form Winkel-Zeit-Diagramm für Beugung und Strekkung und ein Fourier-Spektrum für Beugung und Streckung. Das dargestellte Fourier-Spektrum gibt eine Aussage über die Frequenzanteile, die bei Winkeländerungen der Haltevorrichtung 12 bei der Messung auftreten, d. h die vom Verdrehungswinkel abhängigen und vom statistischen bzw. dynamischen Betrieb abhängigen überlagerten Meßwertschwankungen, die relativ zum jeweiligen bzw. momentanen Meßzustand existieren, können vorzugsweise im Sinne einer Fourier-Analyse ausgewertet werden.

Bezugszeichenliste

10 Vorrichtung
11 Gestell
12 Haltevorrichtung
120 Steg
121 Quersteg
122 Schlitz
123 Handgriff
124 Feststellmittel
125 Platte
126 Einrichtung zum Festhalten der Extremität
14 Achse
15 Antriebsmotor
16 Getriebe
17 Kupplungseinrichtung
170 Leitung
171 Öffner
172 Relais
173 Transistor
174 Optokoppler
176 Schließer
177 Kondensator
178 Widerstand
179 Diode
18 Zahnrad
19 Zahnrad
20 Antriebsachse
21 Zahnriemen
22 Meßwinkelaufnehmeeinrichtung
23 Einrichtung zur Aufnahme des Drehmoments
231 Verstärker
232 Tiefpaßfilter
233 Verstärker
234 Analog-Digital-Wandler
24 Rechner
25 Aufzeichnungsmittel
26 Anzeigemittel
27 Ansteuerelektronikeinrichtung
28 Bit-Rate-Multitlier
29 Monoflop
30 Gatter

**Patentansprüche**

1. Vorrichtung zur Erfassung und Verbesserung der Funktionsfähigkeit des Bewegungsapparates von Menschen, insbesondere seiner Extremitäten, mit einer der Aufnahme der Extremität dienende Haltevorrichtung (12), die durch einen Antriebsmotor (15) um eine in der Haltevorrichtung (12) aufgenommene Gelenkachse einer Extremität (13) entsprechenden Achse (14) drehbar ist dadurch gekennzeichnet, daß die Antriebsachse (20) mit einer Meßwinkelaufnehmereinrichtung (22) sowie einer Einrichtung (23) zur Aufnahme des Drehmomentes versehen ist.

2. Vorrichtung nach Anspruch 1, dadurch gekennzeichnet, daß der Antriebsmotor (15) ein Schrittmotor ist, der über ein Getriebe (16) mit der Achse (14) der Haltevorrichtung (12) verbunden ist.

3. Vorrichtung nach Anspruch 1 oder 2, dadurch gekennzeichnet, daß zwischen Getriebe (16) und der Achse (14) der Haltevorrichtung (12) eine Kupplungseinrichtung (17) angeordnet ist, die bei einem frei wählbaren höchstzulässigen Drehmoment auslösbar ist.

4. Vorrichtung nach einem der Ansprüche 1 bis 3, dadurch gekennzeichnet, daß die Achse (14) der Haltevorrichtung (12) mit der Antriebsachse (20) über eine Zahnradanordnung (18, 19) und unter Zwischenschaltung eines Zahnriemens (21) miteinander verbunden ist.

5. Vorrichtung nach einem der Ansprüche 1 bis 4, dadurch gekennzeichnet, daß der Drehwinkel der Haltevorrichtung (12) über den Antriebsmotor (15) steuernde Taktimpulse erfaßbar ist.

6. Vorrichtung nach einem der Ansprüche 1 bis 5, dadurch gekennzeichnet, daß eine den tatsächlichen Drehwinkel des Gelenks einer Extremität erfassende Winkelaufnehmereinrichtung unmittelbar auf der Extremität angebracht ist.

7. Vorrichtung nach einem der Ansprüche 1 bis 6, dadurch gekennzeichnet, daß die Bewegung der Haltevorrichtung (12) über den Antriebsmotor (15) durch einen zugeordneten Rechner (24) in Abhängigkeit programmierbarer Bewegungs- und Drehmomentsparameter steuerbar ist.

8. Vorrichtung nach einem der Ansprüche 1 bis 7, dadurch gekennzeichnet, daß die bei der Bewegung der Haltevorrichtung (12) um ihre Achse (14) auftretenden Kräfte durch Reibung, Schwerkraft und Trägheit durch den Rechner (24) erfaßt und nachfolgend zur Darstellung der für die Bewegung der Haltevorrichtung (12) typischen tatsächlichen Bewegungsparameter einer Extremität (13) minimalisiert werden.

9. Vorrichtung nach Anspruch 8, dadurch gekennzeichnet, daß die Haltevorrichtung (12) vor der Aufnahme einer Extremität (13) einen Bewegungszyklus zur Ermittlung einer durch Reibung, Schwerkraft und Trägheit der Haltevorrichtung (12) bedingten Eichkurve durchläuft und über den Rechner (24) erfaßbar ist.

10. Vorrichtung nach Anspruch 8 oder 9, dadurch gekennzeichnet, daß die ermittelten Bewegungsparameter wie das Drehmoment der Haltevorrichtung (12) als Funktion dieses Verdrehungswinkels, der zeitabhängige Verdrehungswinkel als Funktion eines Drehimpulses sowie die Verdrehungsarbeit und Leistung ermittelbar und über Aufzeichnungsmittel (25) aufzeichenbar sind.

11. Vorrichtung nach Anspruch 10, dadurch gekennzeichnet, daß die Bewegungsparameter für jede Drehrichtung der Haltevorrichtung (10) gesondert ermittelbar und über Aufzeichnungsmittel (25) aufzeichenbar sind.

12. Vorrichtung nach einem der Ansprüche 8 bis 11, dadurch gekennzeichnet, daß die ermittelten Bewegungsparameter durch Anzeigemittel (26) darstellbar sind.

13. Vorrichtung nach einem der Ansprüche 1 bis 12, dadurch gekennzeichnet, daß im Zuge eines Bewegungsablaufes kurzzeitige Schwankungen einzelner Meßparameter das Drehmoment und nach dem Prinzip der Fourier-Analyse über den Rechner (24) erfaßbar und analysierbar sind.

14. Vorrichtung nach einem der Ansprüche 1 bis 13, dadurch gekennzeichnet, daß die Meßwerte beeinflußende Eigenmasse bzw. das Eigengewicht der bewegten Teile der Exetremitäten bei der Ermittlung von deren Bewegungsparameter im Rechner (24) berücksichtigbar ist.

15. Vorrichtung nach Anspruch 14, dadurch gekennzeichnet, daß die Eigenmasse des Gelenks mittels eines zeitlich sinusförmig ablaufenden Test-Schwingungsvorgangs des Gelenks ermittelbar ist.

## Claims

1. Device for determining and improving the efficiency of the locomotor system of humans, in particular its extremities, comprising a holding device (12) which serves to accommodate the extremity and which can rotate by means of a drive motor (15) about a shaft (14) corresponding to a joint axis, accommodated in the holding device (12), of an extremity (13), characterized in that the drive shaft (20) is provided with a measured angle sensing device (22) and a torque sensing device (23).

2. Device according to claim 1, characterised in that the drive motor (15) is a stepping motor which is connected via a gear unit (16) to the shaft (14) of the holding device (12).

3. Device according to claim 1 or 2, characterised in that a coupling device (17) is arranged between the gear unit (16) and the shaft (14) of the holding device (12) and can be disengaged when the torque is of a maximum permissible value which can be freely selected.

4. Device according to one of claims 1 to 3, characterised in that the shaft (14) of the holding device (12) is connected to the drive shaft (20) via a gearwheel arrangement (18, 19) and with the interposition of a toothed belt (21).

5. Device according to one of claims 1 to 4, characterised in that the angle of rotation of the holding device (12) can be determined by clock pulses which control the drive motor (15).

6. Device according to one of claims 1 to 5, characterised in that an angle sensing device which determines the actual angle of rotation of the joint of an extremity is mounted directly on the extremity.

7. Device according to one of claims 1 to 6, characterised in that the movement of the holding device (12) can be controlled via the drive motor (15) by an associated computer (24) as a function of programmable movement and torque parameters.

8. Device according to one of claims 1 to 7, characterised in that the forces due to friction, gravity and inertia occurring upon the movement of the holding device (12) about its shaft (14) are determined by the computer (24) and subsequently minimised for representing the actual movement parameters, which are typical of the movement of the holding device (12), of an extremity (13).

9. Device according to claim 8, characterised in that, before receiving an extremity (13), the holding device (12) runs through a movement cycle for establishing a calibration curve dependent upon the friction, gravity and inertia of the holding device (12) and can be determined by the computer (24).

10. Device according to claim 8 or 9, characterised in that it is possible to establish the movement parameters, such as the torque of the holding device (12), as a function of this angle of twist, the time-dependent angle of twist as a function of an angular momentum, as well as the amount of twist and power, and to record these by recording means (25).

11. Device according to claim 10, characterised in that the movement parameters for each direction of rotation of the holding device (12) can be established separately and recorded by recording means (25).

12. Device according to one of claims 8 to 11, characterised in that the established movement parameters can be represented by display means (26).

13. Device according to one of claims 1 to 12, characterised in that during a sequence of movements brief variations in individual measurement parameters the torque and can be determined and analysed according to the principle of the Fourier analysis.

14. Device according to one of claims 1 to 13, characterised in that the dead weight of the moving parts of the extremities, which influences the measured values when their movement parameters are established, can be taken into account in the computer (24).

15. Device according to claim 14, characterised in that the dead weight of the joint can be established by means of a joint test procedure using sinusoidal vibration.

**Revendications**

1. Dispositif pour la recherche et l'amélioration de la capacité fonctionnelle de l'appareil locomoteur des êtres humains, en particulier, de ses extrémités, cet appareil comportant un dispositif de retenue (12) servant à recevoir l'extrémité, pouvant tourner, au moyen d'un moteur d'entraînement (15), autour d'un axe (14) correspondant à l'axe d'articulation d'une extrémité (13), venant se loger dans le dispositif de retenue (12), caractérisé en ce que l'axe d'entraînement (20) est pourvu d'un dispositif (22) enregistrant l'angle de mesure, ainsi que d'un dispositif (23) destiné à enregistrer le couple de rotation.

2. Dispositif selon la revendication 1, caractérisé en ce que le moteur d'entraînement (15) est un moteur pas à pas qui est relié, à l'intervention d'un mécanisme (16), à l'axe (14) du dispositif de retenue (12).

3. Dispositif selon la revendication 1 ou 2, caractérisé en ce que, entre le mécanisme (16) et l'axe (14) du dispositif de retenue (12), est monté un dispositif d'accouplement (17) qui peut être déclenché dans le cas d'un couple de rotation maximum autorisé pouvant être sélectionné librement.

4. Dispositif selon une des revendications 1 à 3, caractérisé en ce que l'axe (14) du dispositif de retenue (12) est relié à l'axe d'entraînement (20) à l'intervention d'un système à roues dentées (18, 19) et en intercalant une courroie dentée (21).

5. Dispositif selon une des revendications 1 à 4, caractérisé en ce que l'angle de rotation du dispositif de retenue (12) peut être détecté par des impulsions de rythme commandant le moteur d'entraînement (15).

6. Dispositif selon une des revendications 1 à 5, caractérisé en ce qu'un dispositif enregistreur d'angle détectant l'angle réel de rotation de l'articulation d'une extrémité est adapté directement sur cette dernière.

7. Dispositif selon une des revendications 1 à 6, caractérisé en ce que le mouvement du dispositif de retenue (12) peut être commandé, à l'intervention du moteur d'entraînement (15), par un calculateur (24) qui y est attribué, en fonction des paramètres programmables du mouvement et du couple de rotation.

8. Dispositif selon une des revendications 1 à 7, caractérisé en ce que les forces engendrées lors du mouvement du dispositif de retenue (12) autour de son axe (14) sont déterminées par friction, par la force de la gravité et par inertie au moyen du calculateur (24), tandis qu'elles sont ensuite minimalisées pour représenter les paramètres de mouvements réels d'une extrémité (13), ces paramètres étant spécifiques au mouvement du dispositif de retenue (12).

9. Dispositif selon la revendication 8, caractérisé en ce que, avant de recevoir une extrémité (13), le dispositif de retenue (12) exécute un cycle de mouvement en vue de déterminer une courbe d'étalonnage due au frottement, à la force de la gravité et à l'inertie du dispositif de retenue (12), l'enregistrement pouvant avoir lieu à l'intervention du calculateur (24).

10. Dispositif selon la revendication 8 ou 9, caractérisé en ce que l'on peut déterminer les paramètres de mouvement déterminés tels que le couple de rotation du dispositif de retenue (12) en fonction de cet angle de torsion, l'angle de torsion tributaire du temps comme fonction d'une impulsion de rotation, de même que le travail de torsion et la puissance, ces paramètres pouvant être enregistrés à l'intervention de moyens (25) prévus à cet effet.

11. Dispositif selon la revendication 10, caractérisé en ce que les paramètres de mouvement pour chaque dispositif de rotation du dispositif de retenue (10) peuvent être déterminés séparément et peuvent être enregistrés à l'intervention de moyens (25) prévus à cet effet.

12. Dispositif selon une des revendications 8 à 11, caractérisé en ce que les paramètres de mouvement déterminés peuvent être représentés par des moyens d'affichage (26).

13. Dispositif selon une des revendications 1 à 12, caractérisé en ce que, au cours d'un déroulement d'un mouvement, à l'intervention du calculateur (24), on peut déterminer et analyser des fluctuations de courte durée de différents paramètres de mesure, notamment le couple de rotation et selon le principe de l'analyse de Fourier.

14. Dispositif selon une des revendications 1 à 13, caractérisé en ce que la masse propre ou le poids propre des éléments en mouvement des extrémités, influençant les valeurs de mesure, doit être pris en considération lors de la détermination de leurs paramètres de mouvement dans le calculateur (24).

15. Dispositif selon la revendication 14, caractérisé en ce que la masse propre de l'articulation peut être déterminée au moyen d'un processus d'oscillation d'essai de l'articulation, se déroulant sous forme sinusoïdale dans le temps.

Fig. 1

## Fig. 2

Fig. 3

Fig. 4

# Fig. 5

Taktimpulse

nach 24 {

vorwärts / rückwärts

Ringzähler

27

bipolare Brückenschaltung

30

15   20

# Fig. 6